Europäisches Patentamt

European Patent Office

Office européen des brevets

(11) Publication number: **0 293 079**
**A2**

## (12) EUROPEAN PATENT APPLICATION

(21) Application number: **88303584.2**

(22) Date of filing: **21.04.88**

(51) Int. Cl.⁴: **C12N 15/00 , A61K 39/118 , G01N 33/571 , G01N 33/569 , C12P 21/00**

The applicant has filed a statement in accordance with Rule 28 (4) EPC (issue of a sample only to an expert). Accession number(s) of the deposit(s): ATCC 67677, ATCC 67384.

Claims for the following Contracting States: ES + GR.

(30) Priority: **24.04.87 GB 8709746**

(43) Date of publication of application:
**30.11.88 Bulletin 88/48**

(84) Designated Contracting States:
**AT BE CH DE ES FR GB GR IT LI LU NL SE**

(71) Applicant: **368800 ALBERTA LTD**
**11811 - 91st Avenue**
**Edmonton Alberta(CA)**

(72) Inventor: **Wenman, Wanda Mary**
**11811 - 91st Avenue Edmonton**
**Alberta T6G 1B1(CA)**
Inventor: **Kaul, Ravi**
**82nd Avenue Apt 1102 Edmonton**
**Alberta T6G 0T5(CA)**

(74) Representative: **Sheard, Andrew Gregory et al**
**Kilburn & Strode 30, John Street**
**London WC1N 2DD(GB)**

(54) Chlamydia vaccine.

(57) Chlamydia trachomatis may be used as a source of polypeptides, nucleotide sequences encoding such polypeptides, nucleic acid probes based on the nucleotide sequences encoding such polypeptides, antibodies to such polypeptides and a vaccine for chlamydia, certain components of the vaccine and means for preparing the vaccine and the components.

EP 0 293 079 A2

## CHLAMYDIA VACCINE

### Background

The present invention relates in general to genes and polypeptides of Chlamydia trachomatis - (C. trachomatis) and in particular to: chlamydial polypeptides, nucleotide sequences encoding such polypeptides, nucleic acid probes based on the nucleotide sequences encoding such polypeptides, antibodies to such polypeptides and a vaccine for chlamydia, certain components of the vaccine and methods for preparing the vaccine and the components.

C. trachomatis is a bacterial species implicated in various infectious diseases, including lymphogranuloma venereum, trachoma, inclusion conjunctivitis (blennorrhea) and non-specific urethritis. Infection is generally treated with antibacterials, such as tetacyclines, sulphonamides, erythromycin and chloramphenicol. It would be useful to prepare antigens characteristic of Chlamydia, particularly C. trachomatis, for use as immunological agents in vaccines, as a source of antibodies (monoclonal or polyclonal) and as antigenic components for use in diagnostic and preparative methods.

An extracelluar form of Chlamydia, called the ("elementary body" or "EB"), binds to cells, particularly to epithelial cells. Subsequent to binding, the bacteria may be taken into the cell within a structure called a "phagosome" (a type of cytoplasmic organelle) by the process of endocytosis. On the other hand, in certain cell types, e.g., human B cells and several B lymphocyte cell lines, binding may occur without uptake into the cell. Levitt et al., Immunol. Today, 8, 246-51 (1987)

After being taken up, the fusion of the phagosome with a lysosome is inhibited in permissive cells, such as epithelial cells and fibroblasts. The bacteria organize into a metabolically active form (called a "reticulate body" or "RB") and multiply. Still within a cytoplasmic organelle, the bacteria reorganize into EBs. The EBs are released by cell lysis or exocytosis to infect other cells.

Two proteins of molecular mass ("$M_r$") 18,000 daltons ("18K") and 31,000 daltons ("31K") associated with the infectious EBs of C. trachomatis have been found to bind to eukaryotic cells. Wenman et al., J. Bacteriol., 165, 602-607 (1986); and Hackstadt, J. Bacteriol., 165, 13-20 (1986). These proteins are of particular interest because the attachment of the pathogen to its host cell represents a critical point of interaction and is necessary for successful invasion. Antibodies raised against these chlamydial cell-binding proteins possess neutralizing activity [Wenman et al., in Chlamydial Infections, eds. Oriel et al., Cambridge University Press, 15-18 (1986)] which may indicate a protective role for such antibodies. Moreover, the binding proteins appear to be present on EBs and not on the intracellular and noninfectious RBs. The major binding protein, 18K, is present on C. trachomatis strains, including serovars $L_1$, $L_2$, J and K, and is a surface-exposed component.

Antibodies against C. trachomatis and/or assays containing them are reported in: Numazaki et al., J. Clin. Pathol., 38, 345-350 (1985); Numazaki et al., Tohoku J. Exp. Med., 145, 57-63 (1985); Haukins et al., Br. J. Opthal. Mol., 69, 640-644 (1985); Wang et al., J. Infect. Dis., 152, 791-800 (1985); Zhang et al., J. Immunol., 138, 575-581 (1987); Kiviat et al., JAMA, 253, 997-1000 (1985); and Rasanen et al., Infect. Immun., 54, 28-31 (1986). Barnes et al., J. Clin. Microbiol., 22, 609-613 (1985) describes monoclonal antibodies raised, in part, by injection of EBs. Agabian et al., PCT Publication No. 192,033, describes nucleotide and amino acid sequences of a protein which is immunologically cross-reactive with the major outer membrane protein of C. trachomatis.

### Summary of the Invention

According to the present invention, a chlamydial EB protein or an antigenic subfragment thereof is provided.

Throughout this specification, a "complement" of or a "sequence of nucleotides complementary to" a sequence of nucleotides is intended to encompass all polynucleotides (whether, for example, DNA or RNA) which hybridize with the given sequence under the conditions generally defined in Example 6 below.

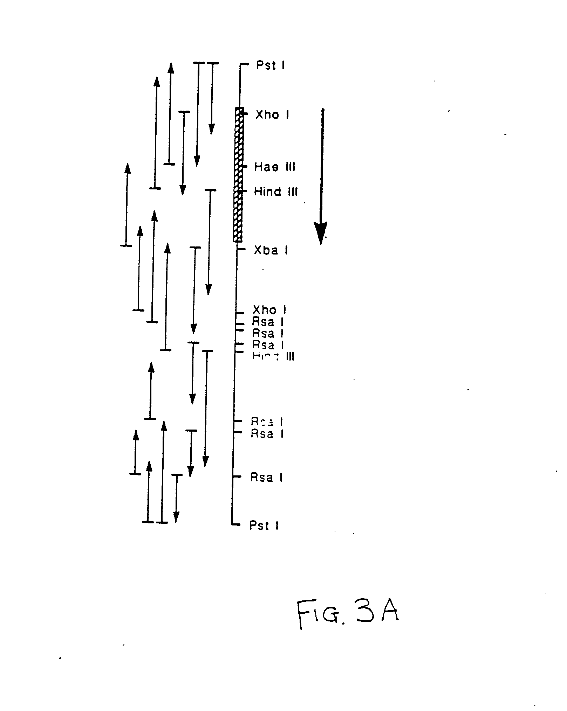

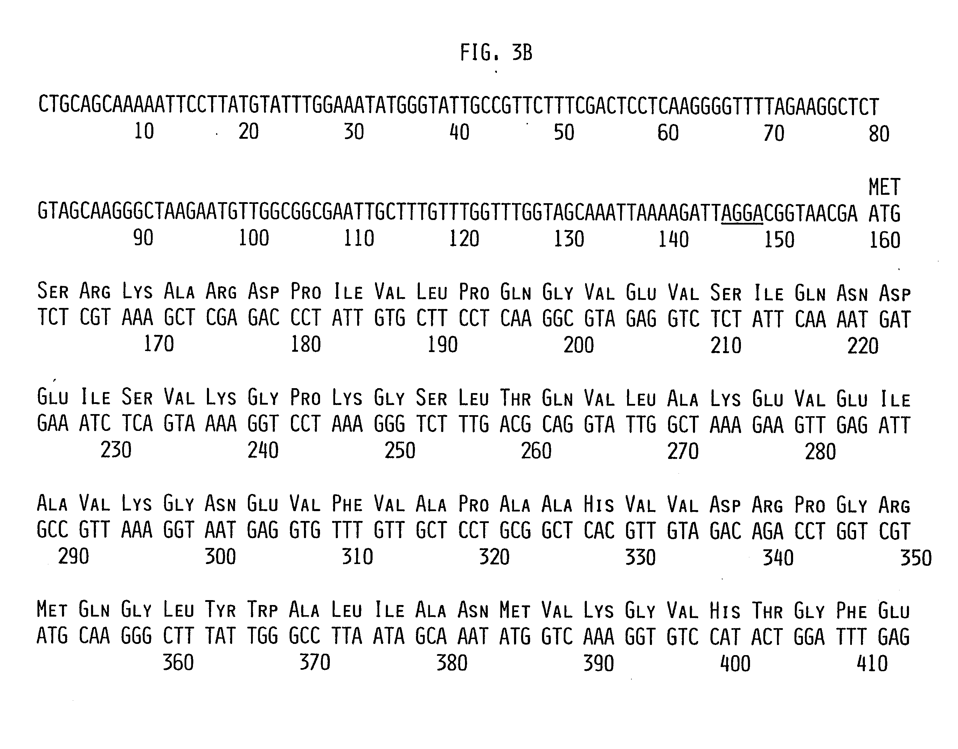

A purified and isolated polypeptide according to the present invention is encoded by a nucleotide sequence selected from the group consisting of: the nucleotide sequence as shown in FIG. 3B; a nucleotide sequence which encodes the same sequence of amino acids as encoded by the nucleotide sequence shown in FIG. 3B; a nucleotide sequence which would hybridize with the complement of any 20 sequential nucleotides as shown in the nucleotide sequence shown in FIG. 3B; a nucleotide sequence which would hybridize with the complement of any 20 sequential nucleotides as shown in the nucleotide sequence shown in FIG. 3B but for the redundancy of the genetic code; and a nucleotide sequence which encodes an epitope encoded by 18 sequential nucleotides in the nucleotide sequence shown in FIG. 3B.

The present invention also provides: a monoclonal or monospecific polyclonal antibody exhibiting a specific immunoreactivity with an antigenic polypeptide according to the present invention, an immortalized cell line producing a monoclonal antibody to the polypeptide; an immunoassay kit comprising an antibody according to the present invention; a method of passive immunization including the step of injection into a patient of such an antibody; a vaccine including a purified and isolated antigenic polypeptide according to the present invention and a pharmaceutically acceptable diluent, adjuvant or carrier; an immunotherapeutic composition including a purified and isolated antibody according to the present invention and a pharmaceutically acceptable diluent adjuvant or carrier. In particular the present invention provides: a vaccine including a purified and isolated 18K C. trachomatis extracellular binding protein substantially free of other C. trachomatis proteins, and a pharmaceutically acceptable diluent, adjuvant or carrier; and a vaccine including a purified and isolated 31K C. trachomatis extracellular binding protein substantially free of other C. trachomatis proteins, and a pharmaceutically acceptable diluent, adjuvant or carrier.

Throughout this specification, the single letter amino acid code may be correlated with corresponding three-letter notation as follows:
D = Asp E = Glu F = Phe K = Lys N = Asn
Q = Gln R = Arg W = Trp Y = Tyr M = Met
C = Cys L = Leu I = Ile H = His T = Thr
S = Ser P = Pro G = Gly V = Val A = Ala


The polypeptide according to the present invention may be substantially pure, by which is meant, for example, at least 50%, 75%, 90%, 95% or 99% or greater by weight pure, in increasing order of preference. The polypeptide may be produced by recombinant DNA techniques and may be referred to as a "recombinant protein." Such a polypeptide may be post-translationally or otherwise modified, for example by glycosylation or methylation.

According to another aspect of the present invention, there is provided a protein identical to or substantially homologous with the sequence

MSRKARDPIVLPQGVEVSIQNDEISVKGPKGSLTQ-
VLAKEVEIAVKGNEVFVAP
AAHVVDRPGRMQGLYWALIANMVKGVHTGFEKR-
LEMIMVASPTGKASAHLHSVL
TSQGIVGDSVEVVTIHKFLKDMRRGRSPVDHPIRA-
SYRKRWMYSCCSRRRRENL

or an antigenic subfragment thereof. In particular the present invention provides: a purified and isolated 18K C. trachomatis extracellular binding protein substantially free of other C. trachomatis proteins; and a purified and isolated 31K C. trachomatis extracellular binding protein substantially free of other C. trachomatis proteins.

Nucleic acid in accordance with the present invention may be cDNA, chemically synthesized DNA, RNA or recombinant DNA.

Preferably the present invention provides a purified and isolated nucleic acid described by a nucleotide sequence selected from the group consisting of: the nucleotide sequence as shown in FIG. 3B; a nucleotide sequence which encodes the same sequence of amino acids as encoded by the nucleotide sequence shown in FIG. 3B; a nucleotide sequence which hybridizes with any 20 sequential nucleotides as shown in the nucleotide sequence shown in FIG. 3B or complements thereof; a nucleotide sequence which would hybridize with the any 20 sequential nucleotides as shown in the nucleotide sequence shown in FIG. 3B of the complement thereof but for the redundancy of the genetic code; and a nucleotide sequence which encodes an epitope encoded by 18 sequential nucleotides in the nucleotide sequence shown in FIG. 3B.

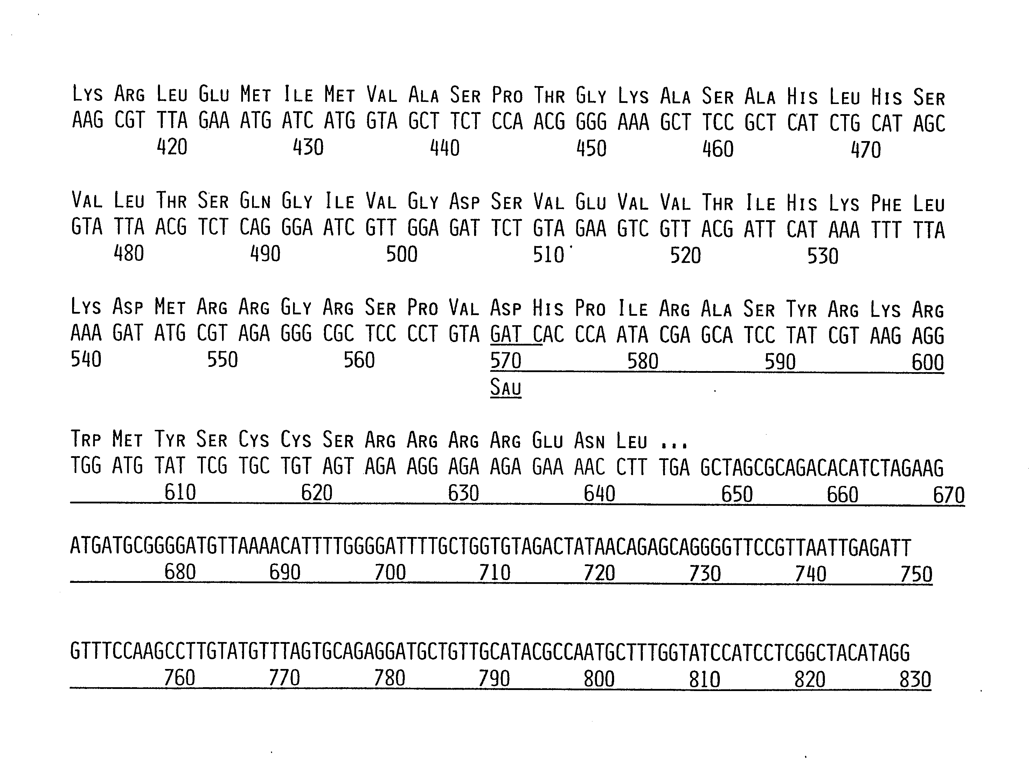

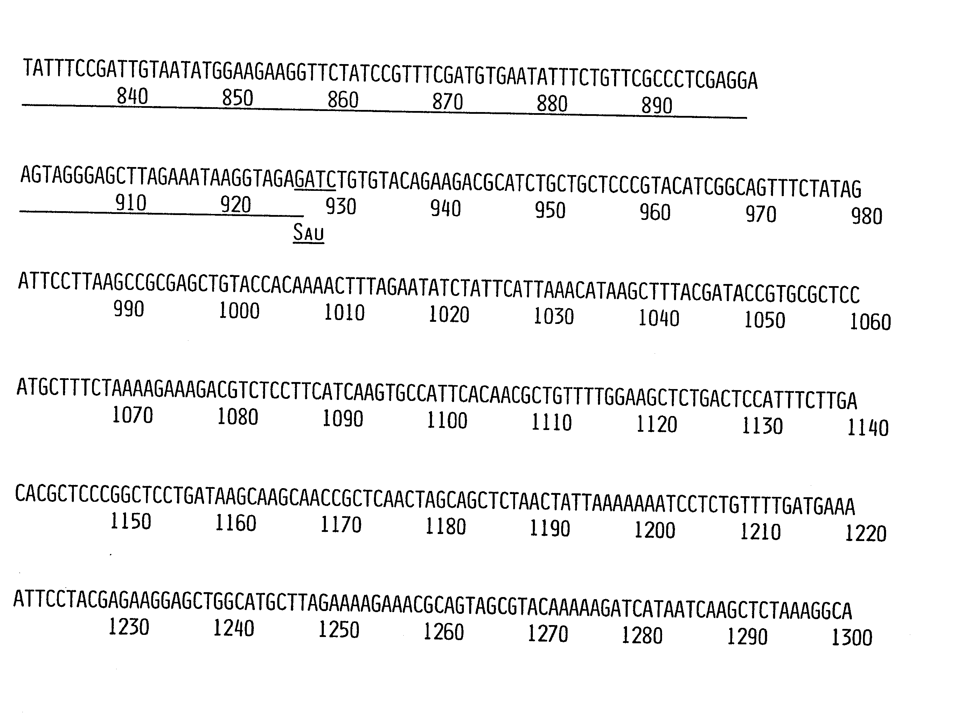

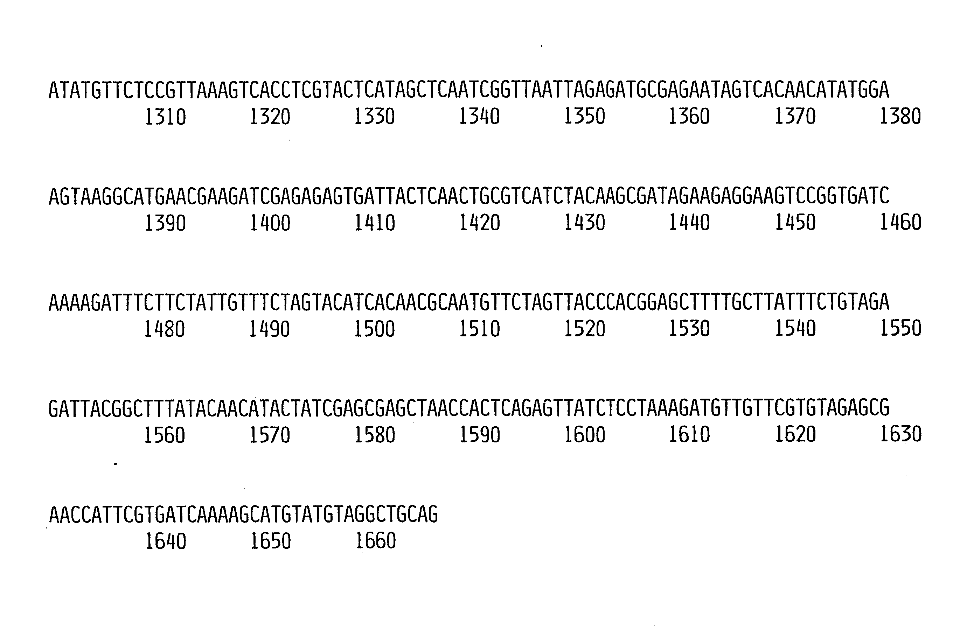

The present invention also provides a cell transformed or transfected with the nucleic acid according to the present invention, preferably a cell of the type deposited as ATCC 67384 or a cell of the type deposited as ATCC 67677. An expression product of a cell and antibodies thereto are also provided according to the present invention. A purified and isolated nucleic acid described by a restriction map shown in FIG. 5, a cell transformed thereby, An expression product of such a cell and antibodies thereto are also provided.

Recombinant DNA in accordance with the invention may be in the form of a vector. According to yet another aspect of the present invention, therefore, there is provided a vector including nucleic acid coding for a protein identical to or substantially homologous with the sequence

MSRKARDPIVLPQGVEVSIQNDEISVKGPKGSLTQ-
VLAKEVEIAVKGNEVFVAP
AAHVVDRPGRMQGLYWALIANMVKGVHTGFEKR-
LEMIMVASPTGKASAHLHSVL
TSQGIVGDSVEVVTIHKFLKDMRRGRSPVDHPIRA-
SYRKRWMYSCCSRRRRENL

or an antigenic subfragment thereof. The vector may be a bacterial plasmid, although other vectors are within the scope of the invention. Suitable bacterial plasmids may be derived from pUC8. A particularly-preferred plasmid has been designated pCT161/18, which has been deposited in a bacterial host on April 9, 1987 as Accession No ATCC 67384 under the terms of the Budapest Treaty at the American Type Culture Collection, 12301 Parklawn Drive, Rockville, Maryland 20852.

Another particularly preferred plasmid according to the present invention contains an insert encoding at least a portion of a 31K EB polypeptide, was designated pCT2/31, and was deposited in a bacterial host E. coli JM83 on 19 April 1988, as Accession No.      at the American Type Culture Collection, 12301 Parklawn Drive, Rockville, Maryland 20852.

A host cell containing a vector in accordance with the present invention itself constitutes still another aspect of the invention. The host cell may be bacterial, but eukaryotic host cells, including yeast cells, are within the scope of the present invention. Bacterial host cells are preferred, especially E. coli, and particularly E. coli JM83/pCT161/18, as deposited in connection with ATCC 67384, and E. coli JM83/pCT2/31, as deposited in connection with ATCC 67677.

According to a further aspect of the invention there is provided a sterile preparation of a protein identical to or substantially homologous with the sequence

MSRKARDPIVLPQGVEVSIQNDEISVKGPKGSLTQ-
VLAKEVEIAVKGNEVFVAP
AAHVVDRPGRMQGLYWALIANMVKGVHTGFEKR-
LEMIMVASPTGKASAHLHSVL
TSQGIVGDSVEVVTIHKFLKDMRRGRSPVDHPIRA-
SYRKRWMYSCCSRRRRENL

or an antigenic subfragment thereof. The sterile preparation may be a vaccine, in which case a physiological carrier, such as physiological saline or phosphate-buffered saline, may also be present. One or more appropriate adjuvants may also be present. Examples of suitable adjuvants include muramyl dipeptide, aluminum hydroxide and saponin. Sterility will generally only be essential if the vaccine is to be administered parenterally.

Brief Description of the Drawings

FIG. 1 is a series of schematic maps of clones according to the present invention;

FIG. 2 is an an illustration of parallel SDS-PAGE gels of polypeptides according to the present invention;

FIG. 3A is a restriction endonuclease map of clone pCT161/18 according to the present invention with arrows showing a nucleotide sequencing strategy; and

FIG. 3B is a nucleotide sequence of chlanectin DNA determined according to the strategy of FIG. 3A and an amino acid sequence deduced from the nucleotide sequence.

Detailed Description

The amino acid sequence of the 18K C. trachomatis binding protein is elucidated according to the present invention, and the protein has been produced by recombinant DNA techniques, including cloning and expression in E. coli. Nucleotide sequences coding for the protein have also been determined. The protein has been named "chlanectin" because of its cell adhesion properties.

Example 1

C. trachomatis serovar L₂ (L2/Bu/434) was grown in HeLa 229 cells and EBs were harvested from HeLa cells after 48 hours and purified as described by Kuo et al. in Non-gonococcal Urethritis and Related Infections, eds. Hobson et al., Am. Soc. Microbiol, 176-185, (1977) Chromosomal DNA was isolated from purified EBs [Wenman et al., Nature, 296, 68-70 (1982); Kaul et al., FEMS Microbiol. Lett., 27, 7-12 (1985)], partially digested with Pst I and sized on a 0.4% agarose gel. DNA fragments (4-6kb) were pooled and ligated to Pst I digested pUC8. E. coli JM-83 cells were used as recipients for transformation experiments [Vieira et al., Gene., 19, 259-268 (1982)]. Approximately 300 colorless ampicillin-resistant recombinants were obtained when grown in the presence of IPTG and X-gal.

Each recombinant colony was grown in L-broth, the cells harvested, suspended in electrophoresis buffer (0.0625 M Tris, 2% SDS, 10% glycerol, 0.001% bromophenol blue and 5% beta-mercaptoethanol) and boiled for 3 minutes. Proteins were separated on 12.5% SDS-PAGE and then either stained with Coomassie blue or transferred to nitrocellulose according to Towbin et al., Proc. Nat'l Acad. Sci. (USA), 46, 4350-4354 (1979). Blots were then reacted with $^{125}$I-labeled HeLa cell membranes as in Wenman et al., J. Bacteriol., 165, 602-607 (1986) [utilizing HeLa cell membranes as well as whole HeLa cells, and see also Hackstadt, J. Bacteriol., 165, 13-20 (1986) using whole HeLa cells rather than HeLa cell membranes]. Isolation of Hela cell membranes and their labeling with $^{125}$I using the lactoperoxidase method is described in Wenman et al., J. Bacteriol., 165, 602-607 (1986).

The E. coli K12 derivative P678-54 [Adler et al., Proc. Nat'l Acad. Sci. (USA), 57, 321-326 (1967)] served as a source of minicells for expression of gene products synthesized in vivo by constructed plasmids. Minicells were constructed in the following manner: competent P678-54 cells were prepared by cold shock treatment using calcium chloride as described by Cohen et al., Proc. Nat'l. Acad. Sci. (USA), 69, 2110-2114 (1972), followed by transformation with pCT161/18. White ampicillin resistant recombinants were identified on IPTG and X-Gal plates. Minicells harboring the appropriate plasmid were isolated by sucrose density gradient centrifugation and labeled with $^{35}$S-methionine [Gill et al., Nature, 282, 797-801 (1979)]. Labeled polypeptides were analyzed by SDS polyacrylamide gel electrophoresis followed by fluorography with ENHANCE (New England Nuclear, Ltd.). In vitro transcription and translation studies were performed using E. coli S-30 extract (Worthington Biochemicals, Freehold, New Jersey), plasmid DNA and $^{35}$S-methionine under the conditions recommended by the manufacturer.

Labeled polypeptides were analyzed by SDS-PAGE and examined by autoradiography. However, for reacting the in vitro-synthesized gene products to $^{125}$I-labeled HeLa cell membranes, $^{35}$S-methionine was deleted from the reaction and replaced by unlabeled methionine and the products were resolved and transferred to nitrocellulose after eletrophoretic separation. The blot was reacted to $^{125}$I-labeled HeLa cell membranes as described in Wenman et al., J. Bacteriol., 165, 602-607 (1986).

HeLa binding and immunoblot assays, used to monitor expression of recombinant polypeptides, were performed on recombinant polypeptides after resolution on SDS-PAGE followed by transfer to nitrocellulose [Towbin et al., Proc. Nat'l Acad. Sci. (USA), 46, 4350-4354 (1979)]. The blots were reacted with $^{125}$I-labeled HeLa cell membranes as described in Wenman et al., J. Bacteriol., 165, 602-607 (1986). An autoradiographic band at 18K was considered positive for HeLa binding assay.

Alternatively, the nitrocellulose blots were reacted with hyperimmune rabbit antiserum to chlanectin (1:100 dilution) and then probed with $^{125}$I-labeled protein A, washed and autoradiographed. An 18 kd polypeptide antigen identified by autoradiography (and its absence in non-expressing recombinants and pUC8 transformed E. coli JM83) was defined as a positive assay. Antisera to purified chlanectin were raised as in Kaul et al., FEMS Microbiol. Lett., 27, 7-12 (1985) with one modification: immunization at weeks two and three was carried out with Freund's incomplete adjuvant rather than complete adjuvant as described in Kaul et al. Chlanectin was purified on 12.5% SDS-PAGE followed by electroelution in phosphate buffered saline at 400 volts for 2 hours.

## Example 2

A recombinant DNA library comprising 4-6 kb fragments of C. trachomatis serovar $L_2$ ($L_2$/Bu/434) DNA was constructed using the expression vector pUC8. From 300 recombinants identified on the basis of ampicillin resistance and insertional inactivation of the lac Z gene, one clone, designated pCT161 encodes a polypeptide ($M_r$ 18,000) identical in size to the chlamydial binding protein. The chimeric pCT161 contains approximately 4.7 kb of C. trachomatis DNA. Complete digestion of pCT161 with the restriction endonuclease Pst I generated 3 fragments, one corresponding to vector DNA and the other two representing the insert. The latter fragments were 3 kb and 1.7 kb in length.

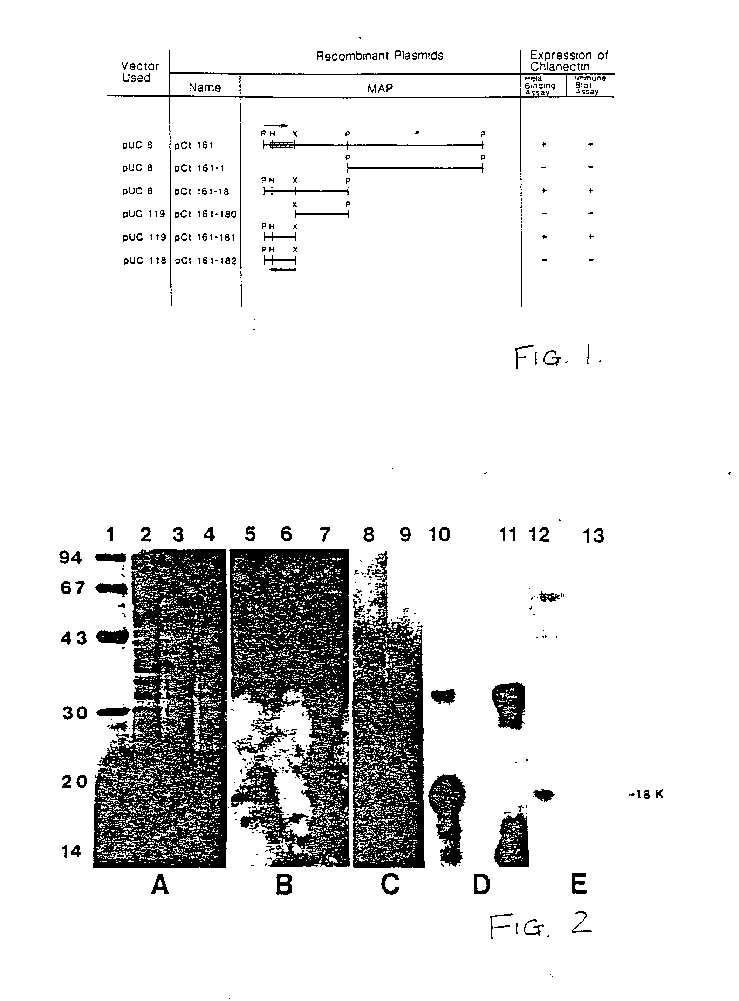

FIG. 1 illustrates the construction of pCT161 chlanectin expression plasmids. A schematic map of sequences and their expression properties is displayed in the figure. Subclones were generated either through single or double digests with restriction endonucleases. For double digestions pCT161/18 was digested with restriction endonucleases Pst1 and Xba1 and the small 667 base pair fragment was resolved on 5% polyacrylamide gels. Following elution from the gel and subsequent ethanol precipitation the DNA was ligated to pUC118 and pUC119 which had been linearized with Pst1 and Xba1. The ligations were incubated overnight at 16°C and then used to transform E. coli JM83 cells. White ampicillin resistant recombinants were identified on IPTG and X-Gal plates.

The expression of the recombinant polypeptides as monitored by their binding to $^{125}$I-labeled HeLa cell membranes or by immunoblot assay using hyperimmune rabbit antisera to chlanectin is indicated on the right in FIG. 1. The cross hatched bar indicates the protein coding region of the chlanectin gene on the cloned DNA insert while the arrow shows the direction of transcription. Only selected restriction sites are shown. In FIG. 1, restriction endonuclease sites are labeled as follows: P = Pst I, H = Hae III, X = Xba I.

Subcloning the individually-isolated Pst1 and Xba1 fragments into pUC8 at the Pst1 restriction site revealed that the 18,000-dalton protein was confined to the small 1.7 kb Pst1 fragment designated as pCT161/18. This subclone, designated pCT161/18 was employed in further experiments.

FIG. 2 illustrates the identification of the chlanectin gene product synthesized by pCT161/18 both in vivo and in vitro. In section A of FIG. 2, a Coomassie blue stained SDS-PAGE gel run according to the procedure set forth in Example 1 on the polypeptides synthesized by plasmid PCT161/18 is shown. Lanes 1-4 represent standard molecular weight markers, pCT161/18 in E. coli K12 strain JM83, pUC8 in JM83 and serovar L2 EB, respectively. In section B of FIG. 2, a gel run in parallel to the gel shown in panel A is depicted after it was reacted with $^{125}$I-labeled HeLa cell membranes; lanes 5-7 Section B of of FIG. 2 are identical, with respect to the amount and type of protein loaded on to each lane, to lanes 2-4. The gel electrophoresis pattern of $^{35}$S-labeled gene products synthesized in minicells prepared as described above, and products synthesized in an in vitro transcription/translation-coupled system are respectively shown in sections C and D FIG 2. The in vitro transcription/translation system is outlined in the protocol provided by the manufacturer Worthington with their transcription/translation kit LS0004401. The reaction contained 1 μg of DNA. Lanes 8 and 10 represent pCT161/18 and lanes 9 and 11 represent pUC8-directed polypeptides. In section E of FIG. 2, a blot of an unlabeled gel run in parallel with the gels of Sections A-D is shown to be similar to panel D in that both products (section E shows results for in vitro transcription/translation products) bind to $^{125}$I-labeled HeLa cell membranes.

Evidence that the recombinant 18K polypeptide is the C. trachomatis binding protein was derived from its binding to $^{125}$I-labeled HeLa cell membranes (Section B of FIG. 2). Labeled HeLa cells also react with native EB adhesins [as reported in: Wenman et al., J. Bacteriol., 165, 602-607 (1986); Hackstadt, J. Bacteriol., 165, 13-20 (1986); and Wenman et al., in: Chlamydial Infections, eds. Oriel et al., Cambridge University Press, 15-18, (1986)] but not with any polypeptides from the host E. coli strain containing pUC8. Thus, pCT161/18 directs synthesis of a protein which possesses the same eukaryotic cell binding determinants as the native chlamydial binding protein.

The plasmids pCT161/18 and pUC8 were introduced into E. coli minicells and the polypeptides they encoded were analyzed. The recombinant pCT161/18 encoded a protein, having an $M_r$ of approximately 18,000, which was absent from minicells containing only pUC8 as indicated in (Section C of FIG. 2).

In vitro synthesis of polypeptides encoded by the plasmid with an E. coli-directed, coupled transcription translation system was used to identify the chlanectin gene product. FIG. 2, section D, shows a gel run on an 18K polypeptide produced using pCT161/18 as compared to the lack of a similar $M_r$ polypeptide using pUC8. Unlabeled products synthesized by this in vitro transcription translation system were found to bind to $^{125}$I-HeLa cells after resolution on SDS-polyacrylamide gels and subsequent ligand blotting, as indicated in FIG. 2, Section E. This suggests that the overall conformation of the protein may not be crucial for eliciting its cell-binding properties.

Example 3

The synthesis of chlanectin by cloned and subcloned DNA fragments was examined by immunoblot assay using hyperimmune rabbit antiserum to the cloned 18K protein as described in Kaul et al., FEMS Microbiol. Lett., 27, 7-12 (1985) with one modification: immunization at weeks two and three was carried out with Freund's incomplete adjuvant rather than complete adjuvant as described in Kaul et al. The recombinant 18K protein was resolved on 12.5% SDS-polyacrylamide gels, identified according to molecular weight standards, but from gels and electroeluted in phosphate buffered saline at 400 volts for 2 hours. Purity was checked by SDS-polyacrylamide gel electrophoresis.

Hyperimmune serum to the purified 18 recombinant polypeptide was raised as described by Kaul et al., FEMS Microbiol. Lett., 27, 7-12 (1985), except that immunization at weeks two and three was performed with Freund's incomplete adjuvant rather than complete adjuvant. This antiserum was also observed to block Chlamydia-HeLa cell association, presumably by binding to chlanectin which then becomes unavailable for subsequent attachment to host cells. Wenman et al., J. Bacteriol., 165, 602-607 (1986). This result supports the use of antichlanectin antibodies in passive immunotherapy as well as the use of recombinant chlanectin in a vaccine.

Example 4

The entire 1658 bp Pst fragment of the plasmid pCT161/18 was sequenced using the dideoxy method with M13 mp18 and M13 mp19 clones.

FIG. 3A shows a restriction endonuclease map of 1658 bp chlamydial DNA encoding chlanectin (pCT161/18). The restriction endonuclease map was generated by single or double digestion with restriction endonucleases as described by Kaul et al., FEMS Microbiol. Lett., 27, 7-12 (1985). The numbers and sizes of the fragments were estimated by electrophoresis on 5% polyacrylamide gels. Positions of sites were confirmed by DNA sequence analysis. The shaded area represents the coding region for the postulated chlanectin protein and open regions show the 3' and 5' non-coding sequence. The arrows below the map indicate a sequencing strategy, the direction and extent of the sequence determined from each cloned DNA fragment being shown by the arrows. The bold arrow above the map indicates direction of transcription.

FIG. 3B shows the complete nucleotide and predicted amino acid sequence of chlanectin DNA. The proposed translation initiation site is the amino acid Met at nucleotides 159-161. The boxed sequences is a postulated Shine-Dalgarno region. Shine et al., Nature, 254, 34-38 (1975). The numbers below each line refer to nucleotide position. The nucleotide sequence was determined by the Sanger dideoxy method using M13 mp18/19. Sanger et al., Proc. Nat'l Acad. Sci. (USA), 74 5463-5468 (1977).

The complete C. trachomatis 18K cell binding protein was located on this 1658 base pair fragment (nucleotides 159-644). The open reading frame encoding the gene may specify a polypeptide of $M_r$ 18,314 comprising 162 amino acids. This is comparable to the reported $M_r$ of 18,000 for the chlamydial binding protein as determined by SDS-PAGE [Wenman et al., J. Bacteriol., 165, 602-607 (1986); Hackstadt, J. Bacteriol., 165, 13-20 (1986); Wenman et al., in Chlamydial Infections, eds. Oriel et al., Cambridge University Press, 15-18 (1986)].

Both the recombinant protein and the native C. trachomatis protein are present on the outer membrane of E. coli and EB respectively, which suggests that leader peptide may be present. Most bacterial proteins which are inserted into the outer membrane have an amino terminal extension with one or more basic residues including at least one arginine or lysine. Michaelis et al., J. Ann. Rev. Microbiol., 36, 435-465 (1982). A small signal sequence of 4-5 amino acids may also be present in chlanectin, which may explain the positively charged amino terminal end and following short hydrophobic region. Similar leader sequences have been described with other proteins. Finlay et al., J. Bacteriol., 165, 625-630 (1986).

The untranslated sequence 10 positions upstream of the ATG start codon contains a sequence AGGA, which probably represents the ribosome binding site [Shine et al., Nature, 254, 34-38 (1975)]. However no sequences similar to the E. coli consensus promoter were found upstream of the Shine-Dalgarno region. Sequence data demonstrated that the chlanectin gene in pCT161/18 is located in the same orientation as the lac promoter of pUC8, but synthesis of a fused gene product was ruled out when stop codons were found in all three reading frames preceding the start codon. To test the dependence of the 18K gene on the lac promoter, the 667 nucleotide Pst I- Xba I fragment was subcloned into the Pst I - Xba I site of pUC118 and pUC119. Expression of a biologically active gene product was obtained with pUC119, where the open reading frame is in same orientation as the lac promoter. However, no expression was obtained with pUC118, which implies the absence of an internal promoter. No other significant open reading frames were found on this fragment. Codon usage for the chlanectin gene is strongly reflective of the low G + C content of C. trachomatis DNA (<50%), with almost 70% of codons favoring U or A in the third position, where a choice was possible.

The cloning and expression of the gene for the functional part of the chlamydial binding protein chlanectin in E. coli facilitates further structural analysis and study of gene product processing. The deduced amino acid sequence of chlanectin, derived from nucleotide sequencing, assists in identifying the domain responsible for cell binding. This domain may be a candidate for future chlamydial vaccine development.

## Example 5

Antisera may be specifically produced by immunizing rabbits with injection of an antigen according to the present invention and an adjuvant, such as Freund's incomplete adjuvant or alum-adsorbed tetanus toxin. Succeeding inoculations may contain the antigen and Freund's incomplete adjuvant. The animals are bled to obtain sera. Polyclonal antibodies may be isolated from the sera by conventional techniques known in the art, Handbook of Experimental Immunology, Vol. 3, Weir, ed., A-3.1 to A-4.10, Blackwell Scientific Publications (1978), which is incorporated by reference herein.

Monoclonal antibodies according to the present invention may be produced according to the procedure of Kohler et al., Nature, 256, 495 (1975) by employing an antigen according to the present invention. Kohler et al. is incorporated by reference herein.

Monoclonal antibodies are produced by injecting mice with immunizing doses of antigen according to the present invention. Spleens are removed from the immunized animals. Spleen cells are fused to myeloma cells using a fusogen, such as polyethylene glycol. Hybridoma cells producing monoclonals are selected in a medium such as HAT medium. Monoclonal antibodies may be isolated from medium in which selected hybridomas have been cultured according to standard procedures known to those skilled in the art.

Example 6

DNA hybridization probes may be synthesized by the procedure of Caruthers, U.S. Patent No. 4,415,732, using the sequence of FIG. 3B, and ribonucleic acid probes may be made by in vitro transcription from them. Hybridization conditions according to the present invention may generally be defined as reactions functionally equivalent to hybridization carried out in 4 × SSC and 0.5% SDS at a temperature of 65° C. in the last wash.

Plasmids, including DNA sequences according to the present invention (e.g. as presented in FIG. 3B), may be labeled with a radioactive isotope [Rigby et al., Mol. Biol., 113, 237-251 (1977) or Feinberg et al., Anal. Biochem., 132 6-13 (1983)] or with a non-radioactive chemical tag [Leary et al., Proc. Nat'l. Acad. Sci. (USA), 80, 4045-4049 (1983)] and used as probes. Such plasmids may also be used to synthesize the labeled RNA probes [Melton et al., Nucleic Acids Res., 12, 7035-7055 (1984)]. The labeled probes may be used to detect the presence of homologous DNA sequences and/or mRNA sequences encoded by these DNA sequences in infected cells either by the Southern or Northern hybridization procedure [Southern et al., J. Mol. Biol., 98, 503 (1975); Thomas, Proc. Nat'l Acad. Sci. (USA), 77, 5201-5205 (1980)] or by dot blot or slot blot hybridization [Kafatos et al., Mol. Cell. Biol., 3, 1097-1107 (1983)], or by in situ hybridization techniques [[Brahic et al., Proc. Nat'l Acad. Sci. (USA), 75, 6125-6129 (1978)].

The recombinant Chlamydia trachomatis gene encoding an 18,000-dalton (18K) protein was utilized as a probe, under the conditions for nick translation with $^{32}$P and hybridization described by Kaul et al., FEMS Microbiol. Lett., 27, 7-12 (1985). The 18K gene was used to probe a C. trachomatis serovar L$_2$ gene bank in vector pUC8 as described in Example 1. The 18K gene hybridized to a recombinant chlamydial gene which encodes a 31,000-dalton protein (31K).

This 31K recombinant C. trachomatis protein may generally be expressed in E. coli strain JM83 on the pUC8 vector by the construction, transformation and expression procedure as set forth in Example 1. One vector constructed for this purpose has been designated pCT2/31. The 31K protein has been initially characterized in the following manner: (a) the recombinant 31K protein binds to $^{125}$I-labeled HeLa cell membranes using a procedure described by Wenman et al., J. Bacteriol., 165, 602-607 (1986). Thus it shares this trait with the native 31K protein; and (b) the recombinant 31K protein is recognized by rabbit antiserum raised against chlamydial EBs and reacted in immunoblots.

Antisera were raised to the recombinant 31K protein as described by Kaul et al., FEMS Microbiol. Lett., 27, 7-12 (1985) and immunoblots were processed following the method of Towbin et al., Proc. Nat'l Acad. Sci. (USA), 76, 4350-4354 (1979). This experiment revealed that the recombinant 31K protein is a better antigen than the native 31K protein, as recognized by immunoblot. Also, in vitro transcription-translation experiments utilizing the recombinant E. coli harbouring the C. trachomatis 31K gene demonstrated a 31K protein unique to that bacterium (not present in E. coli harbouring only vector pUC8). The in vitro transcription-translation experiments were carried out as described in the protocol provided by Worthington for their transcription/translation kit, Catalogue No. LS0004401. The reaction contained 1 μg of DNA.

DNA sequencing Sanger et al., Proc. Nat'l Acad. Sci. (USA), 74, 5463-5467 (1977) has delineated the homologous region between the recombinant 18K and recombinant 31K C. trachomatis genes as indicated by underlining as shown in FIG. 3B. This area of exact base-base matching encompasses the region between the Sau 3A sites at nucleotide 571 and nucleotide 928. The overlap includes both coding and non-coding regions of the 18K gene. The homologous region on 31K gene is not necessarily in reading frame.

The 18K recombinant C. trachomatis protein was localized to the outer membrane of the host E. coli and was isolated and partially purified as described by Kaul et al., J. Bacteriol., 169, 5125-5156 (1987) which is incorporated by references herein. The recombinant 31K protein was identified by a method described by Winkler et al., J. Biological Chemistry, 261, 13838-13843 (1986), the only modification to this published procedure being the use of discontinuous sucrose gradients for centrifugation rather than the 50% glycerol gradients utilized by Winkler.

Example 7

One type of hybridization assay which may be performed using the hybridization probes according to the present invention is called solution hybridization. In this procedure, a labeled probe nucleic acid is added to a solution of a sample to be searched for a target nucleic acid. In order to ensure that both the probe and a target are in a single-stranded state suitable for hybridization, the sample and probe are heated in order to break the hydrogen bonds which are found between complementary strands of a double-stranded probe or a double-stranded target, or which are found within secondary structure of a probe or target. Upon cooling, the reaction is reversed and double-stranded nucleic acid is allowed to form. The amount of double-stranded nucleic acids forms may be determined by scintillation counting of the label on the probe after degradation of unhybridized single strands or after isolating double-stranded DNA by passing the hybridization solution over a hydroxyapatite column which selectively retains the double-stranded form.

In another type of hybridization assay to which the probes according to the present invention may be applied, denatured target nucleic acid is immobilized on a support. Retention of a labeled probe on a support-bound target after passage of the support-bound target through a solution containing the probe permits detection and quantitation of the target by measurement of the amount of bound label. See, e.g., Falkow et al., U.S Patent No. 4,358,535; and Shafritz, European Patent Application No. A1-0062286.

Yet another type of hybridization assay of the present invention in which the probes according to the present invention may be employed is called a "sandwich" hybridization. A two-step sandwich hybridization procedure involves the use of an immobilized target nucleic acid which is exposed in a first step to a first nucleic acid probe having a first portion complementary to the target and having a second portion which is not complementary to the target. In a second step, a second, labeled nucleic acid probe, which is complementary to the second portion of the first probe, is allowed to hybridize to the first probe, forming a "sandwich" comprising the first probe between the target and the second probe. Dunn et al., Cell, 12, 23-36 (1977).

A one-step sandwich assay may also be performed. This type of assay involves the use of a first nucleic acid probe immobilized on a filter. The first nucleic acid probe immobilized on a filter. The first nucleic acid probe is complementary to a fist portion of a target nucleic acid. In a single step the filter-bound first probe is exposed to a sample to be searched for the target nucleic acid sequence and to a second, labeled nucleic acid probe complementary to a second portion of the target nucleic acid which portion is separate from (i.e., non-overlapping with) the portion of the target to which the first probe is complementary. Ranki et al., U.S. Patent No. 4,486,539.

Another approach to hybridization, called blot hybridization involved separating sample nucleic acids according to size by electrophoresis through a gel and then transferring them to a nitrocellulose filter on which they are immobilized in their relative positions on the gel. Because any target in the sample is confined to a distinct band on the filter, even weak signals resulting from small amounts of target may be distinguished from non-specific background after exposure to a radiolabeled probe. Bornkamm et al., Curr. Top. Microbiol. Immunol., 104, 288-298 (1983).

Where a sample is in the form of a touch smear of a fluid, a section through cells, or chromosomal squashes from cells on slides, hybridization may be performed in situ.

A radioactively-labeled probe according to the present invention may be applied to the sample which is bound to the slide in a histological preparation. After coating the slide with a photographic emulsion, autoradiographic procedures reveal the location of target-probe hybrids by means of clusters of silver grains formed in the emulsion over the hybridization site.

Probes according to the present inventor may be constructed based upon the deposits ATCC 67384, ATCC 67677, FIG. 3A, F3B or other information provided herein.

### Example 8

Polyclonal and monoclonal antibodies according to the present invention may be used separately or in combination with purified and isolated antigens according to the present invention in any suitable immunoassay, including immunoblot assays.

Monospecific antiserum was prepared to either the 31K or the 18K recombinant proteins by a modification of the method described by Olmstead, J. Biol. Chem., 256, 11955-11957 (1981). The modification consisted of our use of 1M Tris HCl pH 8.8 to neutralize the eluted material rather than NaOH, as described by Olmstead. This monospecific antiserum may be utilized in neutralization assays as described by Wenman et al. J. Bacteriol., 156, 602-607 (1986).

Target antigens may be adsorbed to polyvinyl titration plates and various dilutions of polyclonal or monoclonal anti-chlanectin antibodies may be applied to the individual wells in a radioimmunoassay [Tsu et al., Selected Methods in Cellular Immunology, Mishell et al., eds, Freeman Publishing Company, San Francisco, 373-397 (1980)].

Immunoassays as described herein may be varied, as is clear to one skilled in the art. Such variations include the use of monoclonal and polyspeific antibodies in conventional and sandwich ELISA assays [Kemeny et al., J. Immunol. Methods, 87, 45-50 (1986)].

### Example 9

Complete and partial chlanectin gene products may be expressed in bacterial, yeast or mammalian expression systems by inserting the DNA sequence of FIG. 3B into a plasmid, a phage or a viral expression vector [Vieira et al., Gene, 19, 259-268 (1982); Young et al., Proc. Nat'l Acad. Sci. (USA), 80, 1194-1198 (1983); Bitter et al., Gene, 32, 263-274 (1984); Cepko et al., Cell, 37, 1053-1062 (1984); and Gorman et al., Mol. Cell. Biol., 2, 1044-1051 (1982)]. The expressed proteins may be purified and used in immunotherapy or to raise specific antbodies.

Example 10

- The DNA sequence of FIG. 3B has been used, as shown therein, to deduce the corresponding protein sequence. Peptides corresponding to different portions of chlanectin proteins, preferably 12-20 amino acid residues in length, may be chemically synthesized by solid-phase methods [Marglin et al., Ann. Rev. Biochem., 39, 841-866 (1970)]. Such peptides may then be used to elicit specific polyclonal and monoclonal antibodies [Lerner, Nature, 299, 592-596 (1982); Niman et al., Proc. Nat'l Acad. Sci. (USA), 80, 4949-4953 (1983)]. The DNA sequence provided in FIG. 3B facilitates the design of immunogenic peptides corresponding to different regions of chlanectin protein, suitable immunogenetic regions of the sequences of FIG. 3B being determined according to procedures known to those skilled in the art [Novotny et al., Proc. Nat'l Acad. Sci. (USA), 83, 226-230 (1980) and Van Regenmortel, Trends Biochem. Sci., 11, 36-39 (1986).

Although the present invention has been described in terms of a preferred embodiment, it is expected that variations and improvements will occur to those skilled in the art upon consideration of the present invention. For example, anti-chlanectin antibodies and fragments thereof may be employed in passive immunization procedures [Beutler et al., Science, 229, 869-871 (1985)]. Anti-idiotypic antibodies may be raised against anti-chlanectin antibodies [Eichmann et al., CRC Crit. Rev. Immunol., 7, 193-227 (1987)] and employed in immunotherapy and in purification of anti-chlanectin antibodies.

Accordingly, it is intended that the appended claims include all such equivalent variations and improvements which come within the scope of the invention as claimed.

**Claims**

1. A purified and isolated antigen, including without limitation antigens produced by expression of recombinant DNA, chemical synthesized polypeptides, proteolytic fragments or analogs of antigens encoded by a nucleotide sequence selected from the group consisting of:

the nucleotide sequence as shown in FIG 3B;

a nucleotide sequence which encodes the same sequence of amino acids as encoded by the nucleotide sequence shown in FIG 3B;

a nucleotide sequence which would hybridize with the complement of any 20 sequential nucleotides as shown in the nucleotide sequence shown in FIG 3B;

a nucleotide sequence which would hybridize with the complement of any 20 sequential nucleotides as shown in the nucleotide sequence shown in FIG 3B but for the redundancy of the genetic code; and

a nucleotide sequence which encodes an epitope encoded by 18 sequential nucleotides in the nucleotide sequence shown in FIG. 3B.

2. A purified and isolated nucleic acid described by a nucleotide sequence selected from the group consisting of:

the nucleotide sequence as shown in FIG 3B;

a nucleotide sequence which encodes the same sequence of amino acids as encoded by the nucleotide sequence shown in FIG 3B;

a nucleotide sequence which hybridizes with any 20 sequential nucleotides or the complement thereof as shown in the nucleotide sequence shown in FIG 3B;

a nucleotide sequence which would hybridize with any 20 sequential nucleotides or the complement thereof as shown in the nucleotide sequence shown in FIG 3B but for the redundancy of the genetic code; and

a nucleotide sequence which encodes an epitope encoded by 18 sequential nucleotides in the nucleotide sequence shown in FIG 3B;

3. A purified and isolated nucleic acid described by the restriction map shown in FIG 5.

4. A cell transformed or transfected with a nucleic acid as claimed in claim 2 or 3.

5. A cell as claimed in claim 4 wherein said cell is of the type deposited as ATCC 67384.

6. An expression product of a cell as claimed in claim 4 or 5.

7. A transformation or transfection vector comprising a nucleic acid as claimed in claim 2.

8. A vector as claimed in claim 7 wherein said vector is pCT161/18.

9. A monoclonal or monospecific polyclonal antibody exhibiting specific immunoreactivity with an antigen as claimed in claim 1 or an expression product as claimed in claim 6.

10. An immortalized cell line capable of producing a monoclonal antibody to an antigen as claimed in claim 1.

11. An immunoassay kit comprising an antibody as claimed in claim 9.

12. The use of an antibody as claimed in claim 9 in the preparation of an immunizing agent.

13. A vaccine and/or sterile preparation comprising:

a purified and isolated antigen as claimed in claim 1; and

a pharmaceutically acceptable diluent, adjuvant or carrier.

14. An immunotherapeutic composition comprising:

a purified and isolated antibody as claimed in claim 9; and

a pharmaceutically acceptable diluent, adjuvant or carrier.

15. A polynucleotide encoding an 18K or 31K C. trachomatis extracellular binding protein substantially free of other C. trachomatis polynucleotides.

16. A transformation or transfection vector comprising a polynucleotide as claimed in claim 15.

17. The vector as recited in claim 16 wherein said vector is pCT2/31.

18. A purified and isolated 18K or 31K C. trachomatis extracellular binding protein substantially free of other C. trachomatis proteins.

19. A vaccine comprising:

a purified and isolated 18K or 31K C. trachomatis extracellular binding protein substantially free of other C. trachomatis proteins; and

a pharmaceutically acceptable diluent, adjuvant or carrier.

Claims for the following Contracting State : ES

1. A process for the production of an antigen corresponding to a nucleotide sequence selected from the group consisting of:

the nucleotide sequence as shown in FIG 3B;

a nucleotide sequence which encodes the same sequence of amino acids as encoded by the nucleotide sequence shown in FIG 3B;

a nucleotide sequence which would hybridize with the complement of any 20 sequential nucleotides as shown in the nucleotide sequence shown in FIG 3B;

a nucleotide sequence which would hybridize with the complement of any 20 sequential nucleotides as shown in the nucleotide sequence shown in FIG 3B but for the redundancy of the genetic code; and

a nucleotide sequence which encodes an epitope encoded by 18 sequential nucleotides in the nucleotide sequence shown in FIG 3B, the process comprising expressing such a nucleotide, chemically synthesising a polypeptide or proteolytically cleaving a larger molecule.

2. A process for the preparation of a nucleic acid described by a nucleotide sequence selected from the group consisting of:

the nucleotide sequence as shown in FIG 3B;

a nucleotide sequence which encodes the same sequence of amino acids as encoded by the nucleotide sequence shown in FIG 3B;

a nucleotide sequence which hybridizes with any 20 sequential nucleotides or the complement thereof as shown in the nucleotide sequence shown in FIG 3B;

a nucleotide sequence which would hybridize with any 20 sequential nucleotides or the complement thereof as shown in the nucleotide sequence shown in FIG 3B but for the redundancy of the genetic code; and

a nucleotide sequence which encodes an epitope encoded by 18 sequential nucleotides in the nucleotide sequence shown in FIG 3B; the process comprising coupling successive nucleotides and/or oligonucleotides together.

3. A process for the preparation of a nucleic acid described by the restriction map shown in FIG 5, the process comprising coupling successive nucleotides and/or oligonucleotides together.

4. A process for preparing a transformed or transfected cell, the process comprising transforming or transfecting a cell with a nucleic acid as claimed in claim 2 or 3.

5. A process as claimed in claim 4 wherein said cell is of the type deposited as ATCC 67384.

6. A process for preparing an expression product of a cell, the process comprising culturing a cell preparable by a process as claimed in claim 4 or 5 under expression conditions.

7. A process for preparing a transformation or transfection vector the process comprising inserting a nucleic acid preparable by a process as claimed in claim 2 into a suitable vector.

8. A process as claimed in claim 7 wherein said vector is pCT161/18.

9. A process for preparing a monoclonal or monospecific polyclonal antibody exhibiting specific immunoreactivity with an antigen preparable by a process as claimed in claim 1 or an expression product preparable by a process as claimed in claim 6, the process comprising harvesting antibodies from hybridoma cells or an immunised animal.

10. A process for the preparation of an immortalized cell line capable of producing a monoclonal antibody to an antigen produceable by a process as claimed in claim 1, the process comprising fusing an antibody-producing cell to an immortal cell.

11. An immunoassay kit comprising a monoclonal or monospecific polyclonal antibody exhibiting specific immunoreactivity with an antigen preparable by a process as claimed in claim 1 or an expression product preparable by a process as claimed in claim 6.

12. The use of an antibody preparable by a process as claimed in claim 9 in the preparation of an immunizing agent.

13. A process for the preparation of a vaccine and/or sterile preparation, the process comprising admixing:

a purified and isolated antigen preparable by a process as claimed in claim 1; and

a pharmaceutically acceptable diluent, adjuvant or carrier.

14. A process for the preparation of an immunotherapeutic composition, the process comprising admixing:

a purified and isolated antibody produceable by a process as claimed in claim 9; and

a pharmaceutically acceptable diluent, adjuvant or carrier.

15. A process for the preparation of a polynucleotide encoding an 18K or 31K C. trachomatis extracellular binding protein substantially free of other C. trachomatis polynucleotides, the process comprising coupling together successive nucleotides and/or oligonucleotides.

16. A process for the preparation of transformation or transfection vector comprising ligating vector nucleic acid with a polynucleotide encoding an 18K or 31K C. trachomatis extracellular binding protein.

17. A process as claimed in claim 16 wherein said vector is pCT2/31.

18. A process for the preparation of purified and isolated 18K or 31K C. trachomatis extracellular binding protein substantially free of other C. trachomatis proteins, the process comprising coupling successive amino acids together by peptide bond formation.

19. A process for the preparation of a vaccine, the process comprising admixing:

a purified and isolated 18K or 31K C. trachomatis extracellular binding protein substantially free of other C. trachomatis proteins; and

a pharmaceutically acceptable diluent, adjuvant or carrier.


Claims for the following Contracting State : GR

1. A process for the production of an antigen corresponding to a nucleotide sequence selected from the group consisting of:

the nucleotide sequence as shown in FIG 3B;

a nucleotide sequence which encodes the same sequence of amino acids as encoded by the nucleotide sequence shown in FIG 3B;

a nucleotide sequence which would hybridize with the complement of any 20 sequential nucleotides as shown in the nucleotide sequence shown in FIG 3B;

a nucleotide sequence which would hybridize with the complement of any 20 sequential nucleotides as shown in the nucleotide sequence shown in FIG 3B but for the redundancy of the genetic code; and

a nucleotide sequence which encodes an epitope encoded by 18 sequential nucleotides in the nucleotide sequence shown in FIG 3B, the process comprising expressing such a nucleotide, chemically synthesising a polypeptide or proteolytically cleaving a larger molecule.

2. A purified and isolated nucleic acid described by a nucleotide sequence selected from the group consisting of:

the nucleotide sequence as shown in FIG 3B;

a nucleotide sequence which encodes the same sequence of amino acids as encoded by the nucleotide sequence shown in FIG 3B;

a nucleotide sequence which hybridizes with any 20 sequential nucleotides or the complement thereof as shown in the nucleotide sequence shown in FIG 3B;

a nucleotide sequence which would hybridize with any 20 sequential nucleotides or the complement thereof as shown in the nucleotide sequence shown in FIG 3B but for the redundancy of the genetic code; and

a nucleotide sequence which encodes an epitope encoded by 18 sequential nucleotides in the nucleotide sequence shown in FIG 3B;

3. A purified and isolated nucleic acid described by the restriction map shown in FIG 5.

4. A cell transformed or transfected with a nucleic acid as claimed in claim 2 or 3.

5. A cell as claimed in claim 4 wherein said cell is of the type deposited as ATCC 67384.

6. An expression product of a cell as claimed in claim 4 or 5.

7. A transformation or transfection vector comprising a nucleic acid as claimed in claim 2.

8. A vector as claimed in claim 7 wherein said vector is pCT161/18.

9. A monoclonal or monospecific polyclonal antibody exhibiting specific immunoreactivity with an antigen as claimed in claim 1 or an expression product as claimed in claim 6.

10. An immortalized cell line capable of producing a monoclonal antibody to an antigen as claimed in claim 1.

11. An immunoassay kit comprising an antibody as claimed in claim 9.

12. The use of an antibody as claimed in claim 9 in the preparation of an immunizing agent.

13. A process for preparing a vaccine and/or sterile preparation, the process comprising admixing an antigen preparable by a process as claimed in claim 1; and

a pharmaceutically acceptable diluent, adjuvant or carrier.

14. A process for preparing an immunotherapeutic composition, the process comprising:

admixing a purified and isolated antibody as claimed in claim 9; and

a pharmaceutically acceptable diluent, adjuvant or carrier.

15. A polynucleotide encoding an 18K or 31K C. trachomatis extracellular binding protein substantially free of other C. trachomatis polynucleotides.

16. A transformation or transfection vector comprising a polynucleotide as claimed in claim 15.

17. A vector as claimed in claim 16 wherein said vector is pCT2/31.

18. A purified and isolated 18K or 31K C. trachomatis extracellular binding protein substantially free of other C. trachomatis proteins.

19. A process for the preparation of a vaccine comprising:

admixing a purified and isolated 18K or 31K C. trachomatis extracellular binding protein substantially free of other C. trachomatis proteins; and

a pharmaceutically acceptable diluent, adjuvant or carrier.

| Vector Used | Name | Recombinant Plasmids MAP | Expression of Chlanectin Hela Binding Assay | Immune Blot Assay |
|---|---|---|---|---|
| pUC 8 | pCt 161 | P H X    P    P | + | + |
| pUC 8 | pCt 161-1 | P    P | – | – |
| pUC 8 | pCt 161-18 | P H X    P | + | + |
| pUC 119 | pCt 161-180 | X    P | – | – |
| pUC 119 | pCt 161-181 | P H X | + | + |
| pUC 118 | pCt 161-182 | P H X | – | – |

FIG. 1.

1 2 3 4 5 6 7 8 9 10 11 12 13

94
67
43
30
20
14

A B C D E

–18 K

FIG. 2

FIG. 3A

## FIG. 3B

CTGCAGCAAAAATTCCTTATGTATTTGGAAATATGGGTATTGCCGTTCTTTCGACTCCTCAAGGGGTTTTAGAAGGCTCT

     10       20       30       40       50       60       70       80

 MET
GTAGCAAGGGCTAAGAATGTTGGCGGCGAATTGCTTTGTTTGGTTTGGTAGCAAATTAAAAGATTAGGACGGTAACGA ATG

     90     100     110     120     130     140     150     160

SER ARG LYS ALA ARG ASP PRO ILE VAL LEU PRO GLN GLY VAL GLU VAL SER ILE GLN ASN ASP
TCT CGT AAA GCT CGA GAC CCT ATT GTG CTT CCT CAA GGC GTA GAG GTC TCT ATT CAA AAT GAT

      170       180      190      200      210      220

GLU ILE SER VAL LYS GLY PRO LYS GLY SER LEU THR GLN VAL LEU ALA LYS GLU VAL GLU ILE
GAA ATC TCA GTA AAA GGT CCT AAA GGG TCT TTG ACG CAG GTA TTG GCT AAA GAA GTT GAG ATT

     230      240     250     260     270     280

ALA VAL LYS GLY ASN GLU VAL PHE VAL ALA PRO ALA ALA HIS VAL VAL ASP ARG PRO GLY ARG
GCC GTT AAA GGT AAT GAG GTG TTT GTT GCT CCT GCG GCT CAC GTT GTA GAC AGA CCT GGT CGT

   290     300     310     320     330     340     350

MET GLN GLY LEU TYR TRP ALA LEU ILE ALA ASN MET VAL LYS GLY VAL HIS THR GLY PHE GLU
ATG CAA GGG CTT TAT TGG GCC TTA ATA GCA AAT ATG GTC AAA GGT GTC CAT ACT GGA TTT GAG

      360     370     380     390     400     410

0 293 079

```
Lys Arg Leu Glu Met Ile Met Val Ala Ser Pro Thr Gly Lys Ala Ser Ala His Leu His Ser
AAG CGT TTA GAA ATG ATC ATG GTA GCT TCT CCA ACG GGG AAA GCT TCC GCT CAT CTG CAT AGC
        420             430             440             450             460             470


Val Leu Thr Ser Gln Gly Ile Val Gly Asp Ser Val Glu Val Val Thr Ile His Lys Phe Leu
GTA TTA ACG TCT CAG GGA ATC GTT GGA GAT TCT GTA GAA GTC GTT ACG ATT CAT AAA TTT TTA
        480             490             500             510             520             530


Lys Asp Met Arg Arg Gly Arg Ser Pro Val Asp His Pro Ile Arg Ala Ser Tyr Arg Lys Arg
AAA GAT ATG CGT AGA GGG CGC TCC CCT GTA GAT CAC CCA ATA CGA GCA TCC TAT CGT AAG AGG
540         550             560             570             580             590             600
                                                SAU


Trp Met Tyr Ser Cys Cys Ser Arg Arg Arg Arg Glu Asn Leu ...
TGG ATG TAT TCG TGC TGT AGT AGA AGG AGA AGA GAA AAC CTT TGA GCTAGCGCAGACACATCTAGAAG
        610             620             630             640             650         660         670


ATGATGCGGGGATGTTAAAACATTTTGGGGATTTTGCTGGTGTAGACTATAACAGAGCAGGGGGTTCCGTTAATTGAGATT
        680     690         700         710         720         730         740         750


GTTTCCAAGCCTTGTATGTTTAGTGCAGAGGATGCTGTTGCATACGCCAATGCTTTGGTATCCATCCTCGGCTACATAGG
        760     770         780         790         800         810         820         830
```

0 293 079

TATTTCCGATTGTAATATGGAAGAAGGTTCTATCCGTTTCGATGTGAATATTTCTGTTCGCCCTCGAGGA

AGTAGGGAGCTTAGAAATAAGGTAGAGATCTGTGTACAGAAGACGCATCTGCTGCTCCCGTACATCGGCAGTTTCTATAG

SAU

ATTCCTTAAGCCGCGAGCTGTACCACAAAACTTTAGAATATCTATTCATTAAACATAAGCTTTACGATACCGTGCGCTCC

ATGCTTTCTAAAAGAAAGACGTCTCCTTCATCAAGTGCCATTCACAACGCTGTTTTGGAAGCTCTGACTCCATTTCTTGA

CACGCTCCCGGCTCCTGATAAGCAAGCAACCGCTCAACTAGCAGCTCTAACTATTAAAAAAATCCTCTGTTTTGATGAAA

ATTCCTACGAGAAGGAGCTGGCATGCTTAGAAAAGAAACGCAGTAGCGTACAAAAAGATCATAATCAAGCTCTAAAGGCA

ATATGTTCTCCGTTAAAGTCACCTCGTACTCATAGCTCAATCGGTTAATTAGAGATGCGAGAATAGTCACAACATATGGA

AGTAAGGCATGAACGAAGATCGAGAGAGTGATTACTCAACTGCGTCATCTACAAGCGATAGAAGAGGAAGTCCGGTGATC

AAAAGATTTCTTCTATTGTTTCTAGTACATCACAACGCAATGTTCTAGTTACCCACGGAGCTTTTGCTTATTTCTGTAGA

GATTACGGCTTTATACAACATACTATCGAGCGAGCTAACCACTCAGAGTTATCTCCTAAAGATGTTGTTCGTGTAGAGCG

AACCATTCGTGATCAAAAGCATGTATGTAGGCTGCAG